# EUROPEAN PATENT APPLICATION

(11) **EP 2 657 868 A2**
(43) Date of publication of application: **30.10.2013**
(21) Application number: 13165290.1
(22) Date of filing: 25.04.2013
(51) Int. Cl.: G06F 19/00

(54) **Medical alert system**

(30) Priority: 25.04.2012 US 201261638341 P
(71) Applicant: Virginia Mason Medical Center, Seattle, Washington 98101 (US)
(72) Inventor: Forrester, Andrew, Seatttle, WA Washington 98101 (US)
(74) Representative: Eisenführ, Speiser & Partner

(57) **Abstract**

Systems and methods for providing medical identification tags wearable by a person that include barcodes (e.g., Quick Response (QR) barcodes, or the like) to provide personal medical information to users (e.g., first responders) with mobile devices capable of scanning the barcodes. The system also facilitates automatic notifications to relatives or others associated with the wearer of the medical identification tag when the barcode is scanned, which may indicate an emergency condition. Wearers of the medical identification tags may modify or update the stored personal medical information as needed.

## Description

### FIELD OF THE INVENTION

The present invention relates generally to medical alerts, and more particularly, to systems and methods for improving medical response in emergency situations utilizing barcodes or other devices.

### BACKGROUND OF THE INVENTION

The following description includes information that may be useful in understanding the present invention. It is not an admission that any of the information provided herein is prior art or relevant to the presently claimed invention, or that any publication specifically or implicitly referenced is prior art.

Generally, a medical identification (ID) tag is a small emblem or tag worn on a bracelet, neck chain, or on the clothing of a wearer bearing a message that the wearer has an important medical condition that might require immediate attention. The intention is to alert a paramedic, physician, emergency department personnel, or other first responders of the condition even if the wearer is not conscious enough or old enough to explain his or her condition. Some people may carry a wallet card with the same information.

### BRIEF DESCRIPTION OF THE DRAWINGS

Exemplary embodiments are illustrated in the referenced figures. It is intended that the embodiments and figures disclosed herein are to be considered illustrative rather than restrictive.

Figure 1 depicts a prior art medical ID bracelet.

Figure 2 illustrates medical alert system according to an embodiment of the present invention.

Figure 3 illustrates a process for implementing the medical alert system of Figure 2.

Figure 4 depicts an example of a medical ID tag including a barcode in accordance with an embodiment of the present invention.

Figure 5 depicts a user information form for providing various types of information for a user.

Figure 6 illustrates an example of a medical ID barcode configured for use in a sporting event environment.

Figure 7 illustrates an example of a barcode configured for use in locating a lost animal.

Figure 8A illustrates a screen shot of an "in case of emergency" button on a smart phone.

Figure 8B illustrates a screen shot of an example display of the smart phone when a user activates the "in case of emergency" button.

Figure 9 illustrates a medical alert system according to an embodiment of the present invention wherein the system receives communications from a medical device.

Figure 10 illustrates a screen shot of an application executing on a smart phone that provides users with various reminders and notifications relating to medical care.

Figure 11 illustrates a diagram of a hardware environment and an operating environment in which one or more of the computing systems of the present invention may be implemented.

### DESCRIPTION OF THE INVENTION

One skilled in the art will recognize many methods, systems, and materials similar or equivalent to those described herein, which could be used in the practice of the present invention. Indeed, the present invention is in no way limited to the methods, systems, and materials described.

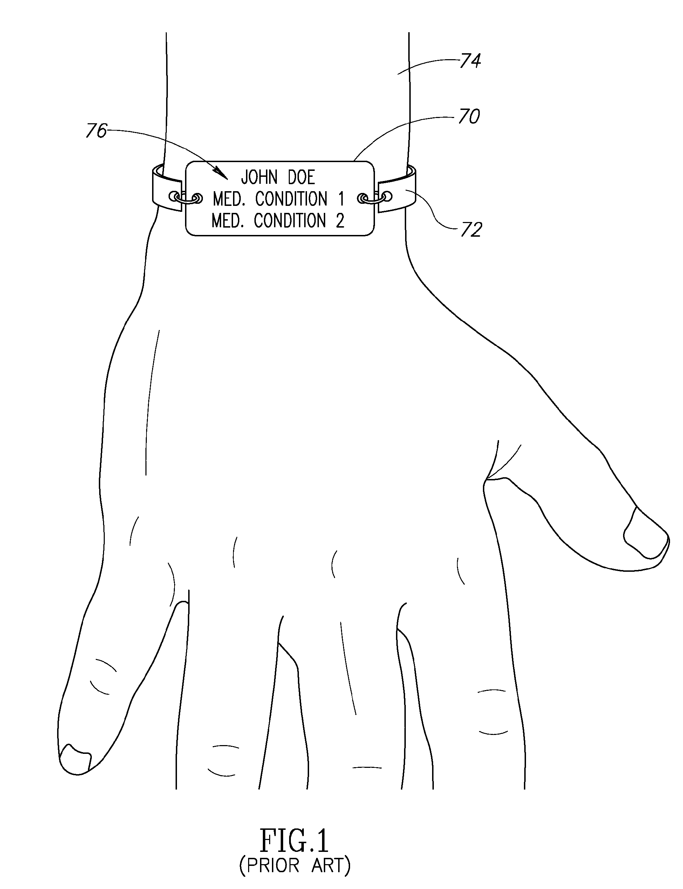

Figure 1 illustrates a medical identification (ID) tag 70 that may be worn on a bracelet 72 of a wrist 74 of a wearer. The ID tag 70 bears a message 76 indicating that the wearer has one or more medical conditions that might require immediate attention. The ID tag 70 provides notice to a paramedic, physician, emergency department personnel, or other first responder of the condition even if the wearer is not conscious enough or old enough to explain his or her medical conditions.

One or more embodiments of the present invention are directed to systems and methods for providing medical identification (ID) tags that include barcodes (e.g., Quick Response (QR) barcodes, or the like) to provide personal medical information to users (e.g., first responders, good Samaritans, etc.) with mobile devices capable of scanning the barcodes. The system also facilitates automatic notifications to emergency contacts (e.g., relatives or others associated with the wearer of the medical identification tag) when the barcode is scanned, which may indicate an emergency condition.

In general, a barcode is an optical machine-readable representation of data, which shows data about the object to which it is attached. As an example, a QR barcode is a matrix barcode (or two-dimensional code) that is readable by QR barcode readers and many currently available camera phones. A QR barcode consists of colored modules arranged in a square pattern on a white background. The information encoded in a QR barcode may be text, a uniform resource locator (URL), or other data. Although QR barcodes are discussed in the description below, it will be appreciated that other types of barcodes (or machine-readable representations of data) may be used as well.

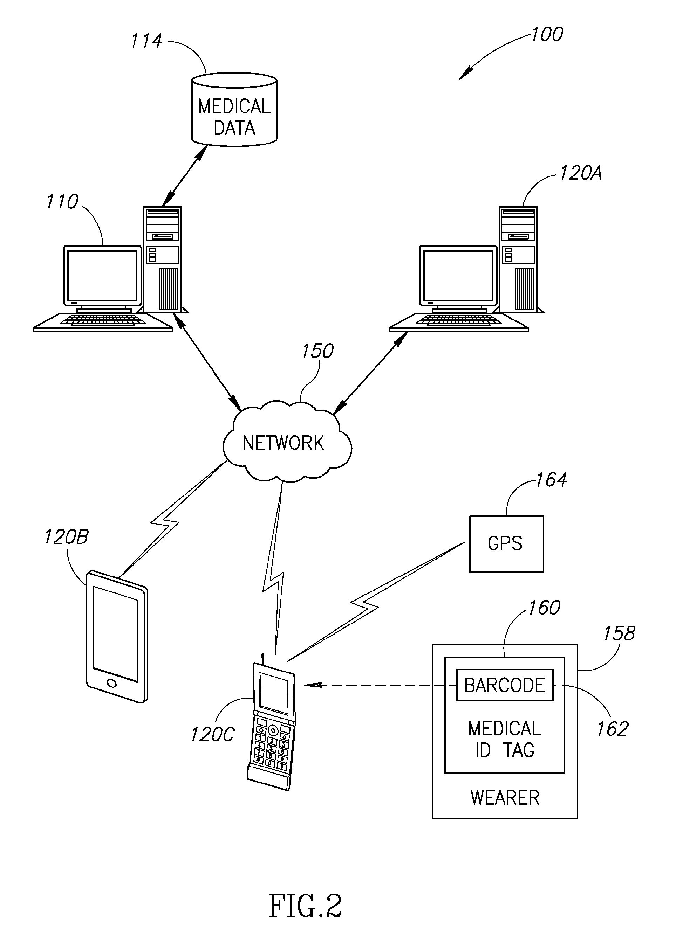

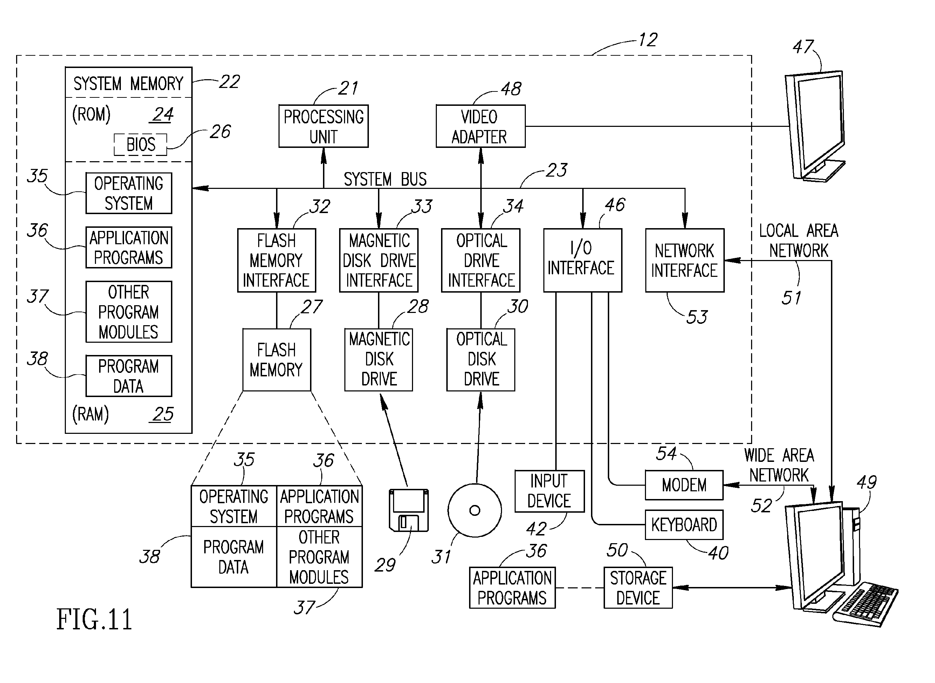

Figure 2 is an illustration of a medical alert system 100 that may be used to provide the functionality of the present invention. The system 100 includes at least one server computing device 110 (e.g., a conventional web server), and at least one client computing device 120 (e.g., client computing devices 120A-120C). The server computing device 110 is connected to the client computing devices 120A-120C by a network 150 (e.g., the Internet). The network 150 may include one or more wireless and/or wired portions. A diagram of a hardware environment and an operating environment in which one or more of the server system 100 and/or client systems 120 may be implemented is shown in Figure 11.

The server computing device 110 is configured to provide one or more of a website, a mobile application, and the like to the client computing devices 120A-120C. It is noted that the various portions of an application may run on the client computing devices 120 and/or the server computing device 110. As can be appreciated, in embodiments in which the server computing device 110 provides a website, the server computing device includes conventional components and related files operable to display a website on the client computing devices 120A-120C. For example, the server computing device 110 may be operative to provide medical data 114 for a wearer or user 158 of a medical ID tag 160 to the client computing devices 120A-120C when one of the devices requests a uniform resource locator (URL) from the server 110 using a web browser by scanning a barcode 162 of the ID tag.

While the system 100 is illustrated as including the single server computing device 110, those of ordinary skill in the art will appreciate that the system 100 may include any number of server computer devices that each perform the functions of the server computing device 110 or cooperate with one another to perform those functions. Further, while the server computing device 110 is illustrated as being connected to the three client computing devices 120A-120C, those of ordinary skill in the art appreciate that the server computing device may be connected to any number of client computing devices and the server computing device is not limited to use with any particular number of client computing devices.

The client computing devices 120A-120C are each operated by a user, such as a first responder in an emergency situation, a friend or family member of the wearer 158, a good Samaritan, etc. The client computing devices 120A-120C may each include a conventional web browser configured to display webpages provided on websites. By way of non-limiting examples, in Figure 2, the client computing device 120A is illustrated as a personal computer (e.g., a laptop, tablet personal computer, and the like), the client computing device 120B is illustrated as a personal digital assistant (PDA), and the client computing device 120C is illustrated as a mobile telephone. The client computing devices 120A-120C may be located remotely from the server computing device 110. The client computing devices 120A-120C also each include an image capturing device (e.g., a camera or scanner) and an application operative to use the image capturing device to read and decode the barcode 162 (e.g., a QR barcode or the like) on the medical ID tag 160.

As shown in Figure 2, each of the client computing devices 120 may be operative to determine its geographic position by communicating with a Global Positioning System (GPS) 164 as known in the art. Using the position information, in some embodiments the notifications or alerts provided to individuals includes the location of the user of the client computing devices 120 at the time the barcode 162 was scanned. The location information may be provided to those receiving an alert or notification so they will know the location of the wearer 158 in need of attention.

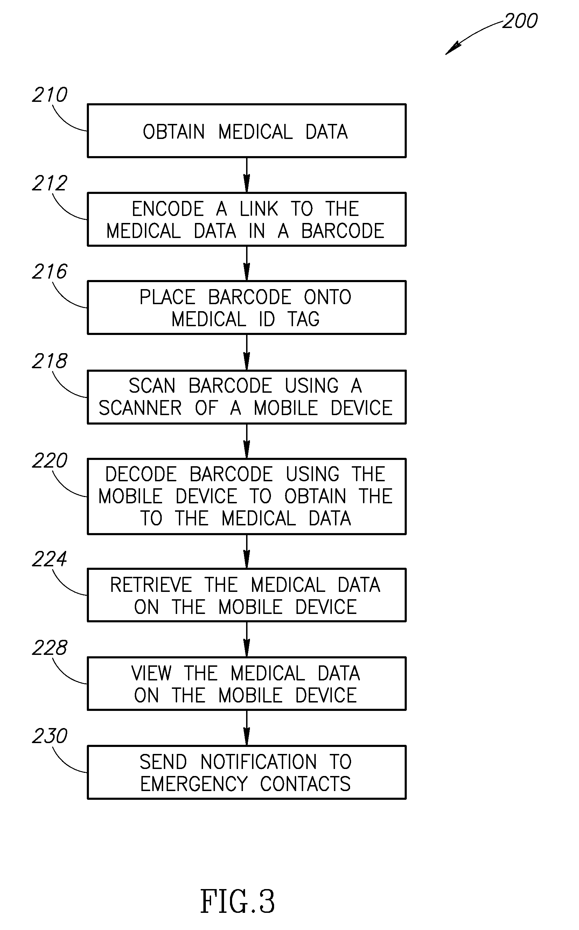

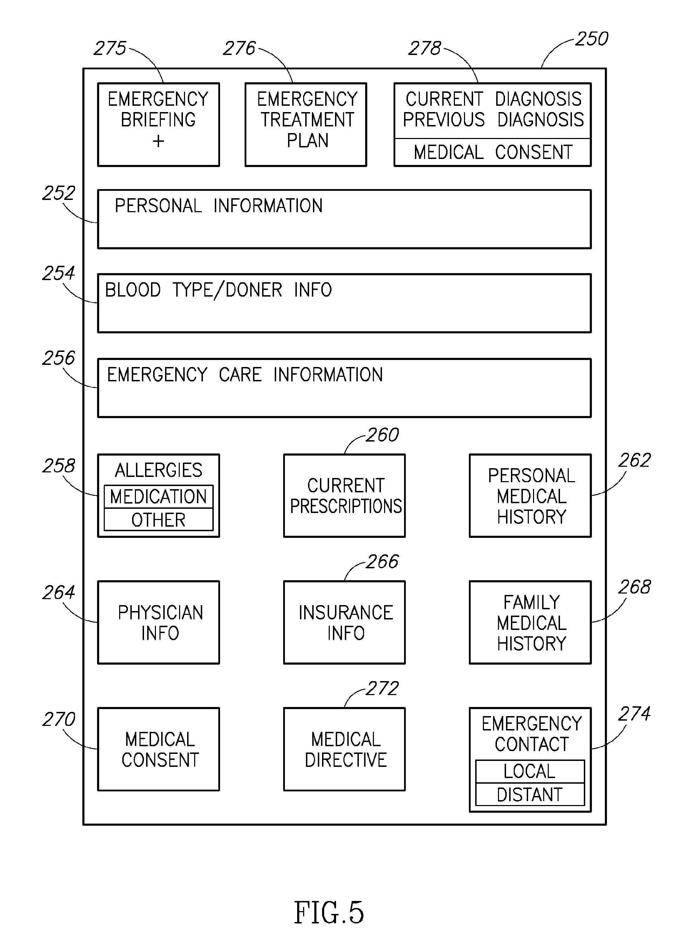

Figure 3 illustrates a flowchart of a process 200 for using barcodes to provide medical data 114 (see Figure 2) to a user, such as a first responder, of one of the client computing devices 120. The example of Figure 3 utilizes QR barcodes, but other types of barcodes may also be used. The process 200 begins at block 210, wherein medical data 114 is obtained for the wearer 158 of the medical ID tag 160. The medical data 114 may include, name, all current diagnoses, emergency treatment plan, blood type, allergies, medical consent, prescriptions, physician info, emergency contacts, organ donor, insurance, medical directive, and other information. The wearer 158 of the medical ID tag 160 may enter his or her medical information in one or more online and/or paper forms. An example of an electronic form that may be used by the wearer 158 or other person to capture the wearer's medical information is shown in Figure 5.

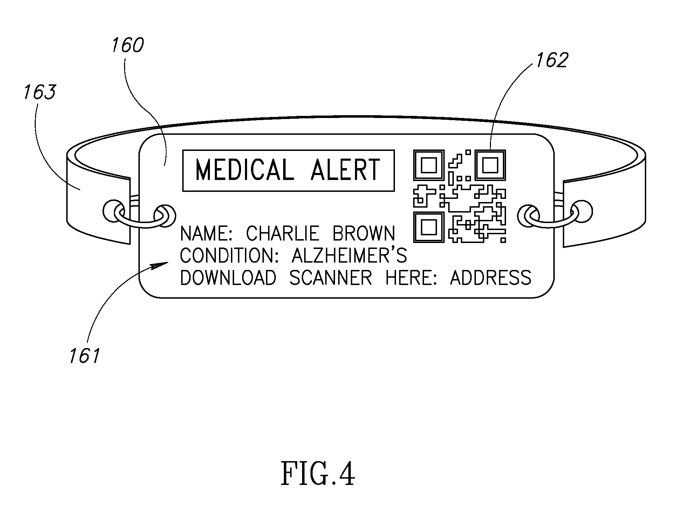

In blocks 212 and 216, a link to the medical data 114 for the wearer 158 is encoded into the barcode 162 (see Figure 2), which is placed on the medical ID tag 160. For example, the link may be in the form of a URL or other suitable link operative to allow one of the client computing devices 120 to access the medical data 114. An example barcode 162 and medical ID tag 160 are shown in Figure 4, wherein the ID tag is coupled to a bracelet 163 that may be worn on the wrist of the wearer 158. Although the medical ID tag 160 shown in Figure 4 comprises the bracelet 163, in other embodiments the medical ID tag comprises a necklace, a wristband, a wallet ID card, a sticker, etc. As shown, the ID tag may include an instruction 161 for a user to obtain a barcode scanner in the case the user does not already have one. As an example, the instructions may direct the user to a website whereat the user can download a suitable barcode scanner onto his or her computing device. In some embodiments, the barcode 162 may be configured for attachment onto an existing medical ID tag. For example, the barcode162 may be formed on stickers that may be coupled to the ID tag 160.

Once the barcode 162 is in place on the medical ID tag 160, a user (e.g., a responder) may then scan the barcode using a scanner of a mobile device (e.g., the consumer devices 120A-120C shown in Figure 2), block 218. The user may scan the barcode 162 at various times, for example, when the wearer 158 of the medical ID tag 160 is in need of medical attention, or at other times. The user's mobile device (e.g., client computing devices 120) will then decode the barcode 162 to obtain the link to the medical data 114, retrieve the medical data 114 over the network 150 (e.g., using a web browser, mobile application, etc.), and view the medical data on the computing device, blocks 220, 224, and 228. Thus, using this method, a user is easily able to use their mobile device to quickly obtain important medical information for the wearer 158 of the medical ID tag 160.

In some embodiments, the system 100 is operative to send automated notifications or alerts to one or more emergency contacts (e.g., family, doctors, etc.) for the wearer 158 in the event the barcode 160 on his or her medical ID tag 160 is scanned, block 230. For example, each of the emergency contacts previously specified by the wearer 158 may receive automatically generated notifications including voice messages, text messages, emails, faxes, etc. In some embodiments, the alerts and/or messages may be customized to include specific messages (e.g., location information, information regarding the person that scanned the barcode 162, etc.). As an example, location information from the mobile device 120 that scanned the barcode 162 may be provided in the notification so the emergency contact will know the location of the wearer. This can be accomplished using the GPS 164 functionality of the client computing devices 120, as described above.

The notifications and alerts may be customized for each recipient or group of recipients. For example, a doctor may receive more technical information than a friend of the wearer 158. This can be configured by the wearer 158 in advance (e.g., via an application or web site). Further, the information provided to the responder that scanned the barcode 162 may be customized to the particular user. For example, the system 100 may be configured to distinguish between particular users (e.g., friends, EMTs, emergency room doctors, etc.) and to provide appropriate information to each user or group of users. The users of the scanner may be identified by an ID on their computing device, or the application executing on the user's computing device may send an identification of the user to the system 100 such that the system recognizes the user that scanned the barcode 162.

In operation, a user (or "patient") that wants to wear the medical ID tag 160 may access the application or web server 110 using his or her computing device 120. Additionally or alternatively, another person such as medical personnel or a relative may access the application or web server 110 on behalf of the wearer of the medical ID tag 160. The user may then create a user account and input his or her medical information 114 into the forms available on the website (see Figure 5). Once the user has entered the relevant medical information, the user may be directed to a "store" to purchase or select one or more types of medical ID tags 160. The system 100 may then order the selected medical ID tags that include a barcode encoded with the user's identity, and send the medical ID tag or tags to the user for wearing. Thereafter, the user may access the application or web server 110 to update or add any additional information as desired. Thus, assuming the wearer keeps his or her medical data 114 current, the information obtained by a first responder by scanning the barcode 170 will be the wearer's current information.

Figure 5 illustrates an example form document or page 250 that may be provided to a wearer of the barcode 162. The page 250 may be provided to a wearer in electronic format (e.g., an application, web site, etc.) or by paper. The page 250 includes a plurality of different types of information that may be provided by the wearer, and which may ultimately be provided to a responder that scans the barcode 162, for example, during a medical emergency. The form page 250 includes a personal information section 252 that allows the user to enter information such as name, address, and telephone. The page 250 also includes a blood type/donor information section 254 where the user can enter their blood type, blood donor information, organ donor information, etc. The page 250 further includes an emergency care information section 256 that allows the user to enter contact information for their doctors or other medical providers. The page 250 also includes an allergies section 258, a current prescriptions section 260, a personal medical history section 262, a physician information section 264, an insurance information section 266, a family medical history section 268, a medical consent section 270, a medical directive section 272, an emergency contact section 274, an emergency briefing section 275, an emergency treatment plan section 276, and a current/previous diagnosis section 278. Some or all of these sections may be provided by the form page 250, as well as other sections not specifically depicted. As can be appreciated, the form page 250 allows users to enter a vast amount of personal and medical information into the system 100. The user may be provided with control of what information is accessible to others using various privacy settings.

In this regard, any user worldwide may access the website, provide personal medical information, and create barcode encoded medical ID tags for use by medical personnel in the case of an emergency and to notify personal contacts in such an emergency. Since the medical ID tag includes a link to the medical information rather than all of the medical information itself, the wearer is able to maintain the privacy of his or her medical information. Further, as can be appreciated, more detailed medical information can be provided to first responders than would practically fit on customary medical ID tags. Additionally, users can update the information by simply updating the form page 250 without requiring a new barcode 162.

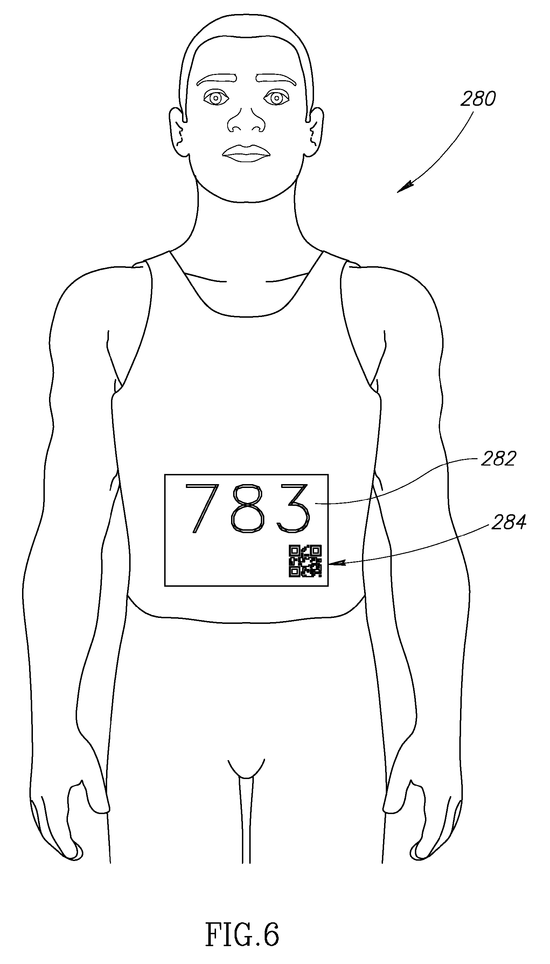

Figure 6 illustrates another application according to an embodiment of the present invention. In this embodiment, an athlete 280 participating in a sporting event wears a bib 282 to identify himself in the event. A barcode 284 may be provided on the bib 282 that encodes a link to medical data 114 (see Figure 2) for the athlete 280. As can be appreciated, sporting events generally involve some level of risk, and it is not uncommon for an athlete to need some type of medical attention during an event. By providing the barcode 284 on the bib 282 of the athlete 280, medical personnel associated with the sporting event quickly have access to the athlete's medical information by simply scanning the barcode 284 with a scanner (e.g., camera of a smart phone). The sporting events may include running races, bicycle races, or any other event where this feature is desired.

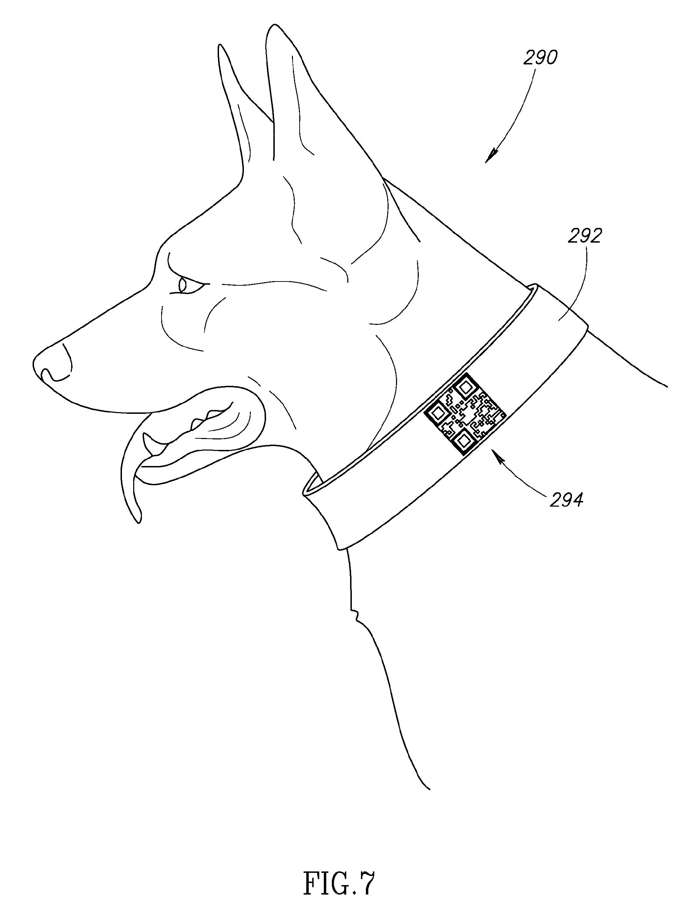

Figure 7 illustrates another application of the system 100 shown in Figure 2. In this embodiment, a barcode 294 is positioned on a collar 292 of a dog 290, and may be used to locate the dog should it become lost. It will be appreciated that the barcode 294 may be positioned elsewhere, such as on a tag of the collar 292. Further, although a dog is shown, it will be appreciated that this embodiment may be used with various types of animals (including humans).

In the embodiment of Figure 7, a pet owner accesses the system 100 and enters various information about the dog 290 in an account. For example, the pet owner may enter his or her contact information, contact information of friends or relatives, veterinarian contact information, and medical information about the dog 290. The barcode 294 is associated with the account such that when the barcode 294 is scanned (e.g., by one of the computing devices 120) by a user that finds the dog, the user is automatically provided with information concerning the dog 290. Using this information, the user may contact the pet owner or others whose information is stored in the system 100.

Similar to other embodiments described above, in the embodiment shown in Figure 7, when a user scans the barcode 294, one or more notifications or alerts may be sent to various people (e.g., the pet owner, a veterinarian, etc.) indicating that the lost dog 290 has been found. The notifications or alerts may also include the location of the user that scanned the barcode 294 by using a suitable location determination system (e.g., the GPS 164 of Figure 2). The pet owner may access the system 100 from time to time and update the information provided as needed. As can be appreciated, this embodiment provides a low cost an useful means for locating lost animals.

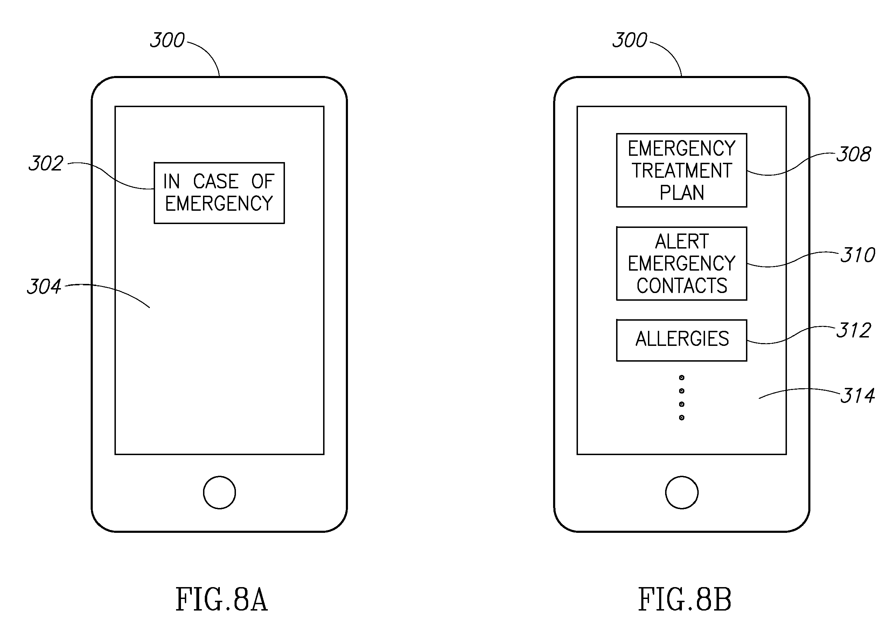

Figures 8A and 8B illustrate another embodiment of the present invention. In this embodiment, rather than using a barcode, the user having an account on the system 100 is provided with an software icon 302 that displays on a display 304 of the user's computing device 300 (e.g., phone). In some embodiments, the icon 302 may be configured to display even when the computing device 300 is "locked." The icon 302 may include words such as "in case of emergency" or other words that would direct a user to activate the icon in a situation when the owner of the computing device 300 needs medical attention. In the event the owner of the computing device 300 is in need of medical attention, a first responder may press the icon 302 on the computing device, which causes a new display 314 to appear that includes various medical information for the owner of the computing device, such as an emergency treatment plan 308, an "alert emergency contacts" icon 310 that will automatically send notifications to others as described above, allergies information 312, etc. Thus, anyone that should happen to find the owner of the computing device 300 in a condition needing medical attention, the responder can access medical information for the user by simply pressing on the icon 302 on the user's computing device 300. Further, the user's contacts (e.g., family, friends, etc.) may be automatically notified almost immediately (e.g., through text message, email, voice message, etc.).

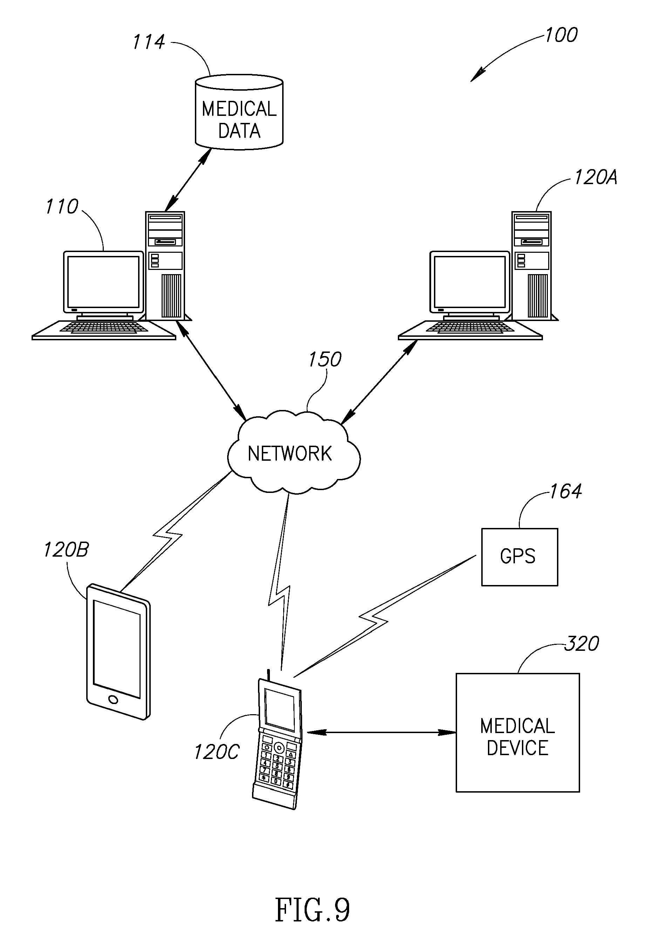

Figure 9 illustrates another embodiment of the present invention. Many of the components of the system 100 shown in Figure 9 are described above with reference to Figure 2. In this embodiment, a medical device 320 is provided that communicates with a user's (or patient's) computing device 120. The medical device 320 may communicate with the computing device 120 using any suitable wired or wireless protocol (e.g. BLUETOOTH®, NFC, WI-FI®, USB, etc.). The medical device 320 may comprise various types of medical devices. Non-limiting examples of medical devices include blood pressure cuffs, glucose readers, pulse oximeters, weight measurement scales, etc.

In operation, the medical device 320 may communicate diagnostic information to the user's computing device 120C, which may in turn be recorded by the system 100 and/or sent to one or more users, as discussed above. For example, in the case where the medical device is a glucose reader, the system may track readings and provide notifications to one or more individuals if the readings are abnormal. In some cases, the data from the medical device 320 may be used to notify the user that he or she should consult with medical personnel. In other cases, the data from the medical device 320 may indicate an emergency condition that requires automatic notification of emergency medical personnel. As discussed above, such notifications may be automatic based on predetermined conditions, and may include location information, the patient's medical information, etc. Further, family or friends of the patient may be notified as desired by the patient.

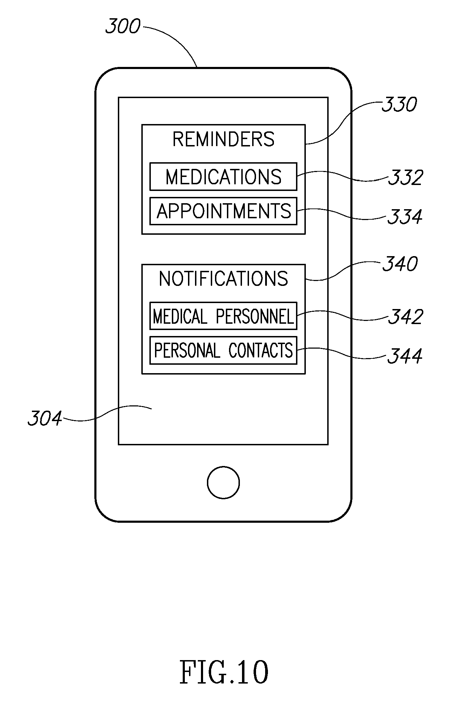

Figure 10 illustrates a screen 304 of a user's computing device 300 for another embodiment of the present invention. The features in this embodiment may be used independently or in conjunction with the embodiments described above. In this embodiment, the user may configure the application (or web site) to provide automatic reminders 330 for medications 332, appointments 334, or other actions (e.g., call doctor). The application may also allow a user to configure notifications 340 that may be sent to medical personnel 342 or personal contacts 344. As discussed above, the user may have full control over what information is sent out and to whom it is sent.

As an example, the user may set up reminders to take one or more medications. The system 100 may require the user to provide an acknowledgment that the medication has been taken (e.g., by selecting an icon, etc.) each time, so that the system can keep track of the users actions. The system 100 may notify the user, medical personnel, or personal contacts if an abnormal condition is detected. Such conditions may include, but are not limited to, missed medications, missed appointments, etc.

As can be appreciated, the systems and methods described herein may be utilized in numerous environments. For example, a company may recommend to its employees that they utilize the system 100. The employees that do so may be incentivized in some way (e.g., lower premiums, gifts, etc.). This may improve the overall health of the employees of the company, which also has many benefits for the company. Those skilled in the art will recognize the numerous other applications in which the systems and methods described herein may be implemented.

Figure 11 is a schematic diagram of hardware and an operating environment in conjunction with which implementations of the medical alert system described above may be practiced. The description of Figure 11 is intended to provide a brief, general description of suitable computer hardware and a suitable computing environment in which implementations of the present invention may be practiced. Although not required, implementations are described in the general context of computer-executable instructions, such as program modules, being executed by a computer, such as a personal computer or the like. Generally, program modules include routines, programs, objects, components, data structures, etc., that perform particular tasks or implement particular abstract data types.

Moreover, those skilled in the art will appreciate that implementations may be practiced with other computer system configurations, including hand-held devices, multiprocessor systems, microprocessor-based or programmable consumer electronics, network PCs, minicomputers, mainframe computers, cloud computing architectures, and the like. Implementations may also be practiced in distributed computing environments where tasks are performed by remote processing devices that are linked through one or more communications networks. In a distributed computing environment, program modules may be located in both local and remote memory storage devices.

The exemplary hardware and operating environment of Figure 11 includes a general-purpose computing device in the form of a computing device 12. The computing device 12 includes the system memory 22, a processing unit 21, and a system bus 23 that operatively couples various system components, including the system memory 22, to the processing unit 21. There may be only one or there may be more than one processing unit 21, such that the processor of computing device 12 comprises a single central-processing unit (CPU), or a plurality of processing units, commonly referred to as a parallel processing environment. The computing device 12 may be a conventional computer, a distributed computer, a mobile computing device, or any other type of computing device.

The system bus 23 may be any of several types of bus structures including a memory bus or memory controller, a peripheral bus, and a local bus using any of a variety of bus architectures. The system memory 22 may also be referred to as simply the memory, and may include read only memory (ROM) 24 and random access memory (RAM) 25. A basic input/output system (BIOS) 26, containing the basic routines that help to transfer information between elements within the computing device 12, such as during start-up, may be stored in ROM 24. The computing device 12 may further include a hard disk drive 27 for reading from and writing to a hard disk, not shown, a magnetic disk drive 28 for reading from or writing to a removable magnetic disk 29, and an optical disk drive 30 for reading from or writing to a removable optical disk 31 such as a CD ROM, DVD, or other optical media. The computing device 12 may also include one or more other types of memory devices (e.g., flash memory storage devices, and the like).

The hard disk drive 27, magnetic disk drive 28, and optical disk drive 30 are connected to the system bus 23 by a hard disk drive interface 32, a magnetic disk drive interface 33, and an optical disk drive interface 34, respectively. The drives and their associated computer-readable media provide nonvolatile storage of computer-readable instructions, data structures, program modules, and other data for the computing device 12. It should be appreciated by those skilled in the art that any type of computer-readable media which can store data that is accessible by a computer, such as magnetic cassettes, flash memory cards, USB drives, digital video disks, Bernoulli cartridges, random access memories (RAMs), read only memories (ROMs), and the like, may be used in the exemplary operating environment. As is apparent to those of ordinary skill in the art, the hard disk drive 27 and other forms of computer-readable media (e.g., the removable magnetic disk 29, the removable optical disk 31, flash memory cards, USB drives, and the like) accessible by the processing unit 21 may be considered components of the system memory 22.

A number of program modules may be stored on the hard disk drive 27, magnetic disk 29, optical disk 31, ROM 24, or RAM 25, including an operating system 35, one or more application programs 36, other program modules 37 (e.g., one or more of the modules and applications described above), and program data 38. A user may enter commands and information into the computing device 12 through input devices such as a keyboard 40 and pointing device 42. Other input devices (not shown) may include a microphone, joystick, game pad, satellite dish, scanner, or the like. These and other input devices are often connected to the processing unit 21 through a serial port interface 46 that is coupled to the system bus 23, but may be connected by other interfaces, such as a parallel port, game port, a universal serial bus (USB), or the like. A monitor 47 or other type of display device is also connected to the system bus 23 via an interface, such as a video adapter 48. In addition to the monitor, computers typically include other peripheral output devices (not shown), such as speakers and printers.

The computing device 12 may operate in a networked environment using logical connections to one or more remote computers, such as remote computer 49. These logical connections are achieved by a communication device coupled to or a part of the computing device 12 (as the local computer). Implementations are not limited to a particular type of communications device. The remote computer 49 may be another computer, a server, a router, a network PC, a client, a memory storage device, a peer device or other common network node, and typically includes many or all of the elements described above relative to the computing device 12. The remote computer 49 may be connected to a memory storage device 50. The logical connections depicted in Figure 11 include a local-area network (LAN) 51 and a wide-area network (WAN) 52. Such networking environments are commonplace in offices, enterprise-wide computer networks, intranets and the Internet.

When used in a LAN-networking environment, the computing device 12 is connected to the local area network 51 through a network interface or adapter 53, which is one type of communications device. When used in a WAN-networking environment, the computing device 12 typically includes a modem 54, a type of communications device, or any other type of communications device for establishing communications over the wide area network 52, such as the Internet. The modem 54, which may be internal or external, is connected to the system bus 23 via the serial port interface 46. In a networked environment, program modules depicted relative to the personal computing device 12, or portions thereof, may be stored in the remote computer 49 and/or the remote memory storage device 50. It is appreciated that the network connections shown are exemplary and other means of and communications devices for establishing a communications link between the computers may be used.

The computing device 12 and related components have been presented herein by way of particular example and also by abstraction in order to facilitate a high-level view of the concepts disclosed. The actual technical design and implementation may vary based on particular implementation while maintaining the overall nature of the concepts disclosed.

The foregoing described embodiments depict different components contained within, or connected with, different other components. It is to be understood that such depicted architectures are merely exemplary, and that in fact many other architectures can be implemented which achieve the same functionality. In a conceptual sense, any arrangement of components to achieve the same functionality is effectively "associated" such that the desired functionality is achieved. Hence, any two components herein combined to achieve a particular functionality can be seen as "associated with" each other such that the desired functionality is achieved, irrespective of architectures or intermedial components. Likewise, any two components so associated can also be viewed as being "operably connected," or "operably coupled," to each other to achieve the desired functionality.

While particular embodiments of the present invention have been shown and described, it will be obvious to those skilled in the art that, based upon the teachings herein, changes and modifications may be made without departing from this invention and its broader aspects and, therefore, the appended claims are to encompass within their scope all such changes and modifications as are within the true spirit and scope of this invention. Furthermore, it is to be understood that the invention is solely defined by the appended claims. It will be understood by those within the art that, in general, terms used herein, and especially in the appended claims (e.g., bodies of the appended claims) are generally intended as "open" terms (e.g., the term "including" should be interpreted as "including but not limited to," the term "having" should be interpreted as "having at least," the term "includes" should be interpreted as "includes but is not limited to," etc.).

It will be further understood by those within the art that if a specific number of an introduced claim recitation is intended, such an intent will be explicitly recited in the claim, and in the absence of such recitation no such intent is present. For example, as an aid to understanding, the following appended claims may contain usage of the introductory phrases "at least one" and "one or more" to introduce claim recitations. However, the use of such phrases should not be construed to imply that the introduction of a claim recitation by the indefinite articles "a" or "an" limits any particular claim containing such introduced claim recitation to inventions containing only one such recitation, even when the same claim includes the introductory phrases "one or more" or "at least one" and indefinite articles such as "a" or "an" (e.g., "a" and/or "an" should typically be interpreted to mean "at least one" or "one or more"); the same holds true for the use of definite articles used to introduce claim recitations. In addition, even if a specific number of an introduced claim recitation is explicitly recited, those skilled in the art will recognize that such recitation should typically be interpreted to mean at least the recited number (e.g., the bare recitation of "two recitations," without other modifiers, typically means at least two recitations, or two or more recitations).

## Claims

1. A computer-implemented method for providing a medical alert system, the method comprising:
providing a database for storing medical information for a first user;
providing the first user with access to the database via a first user computing device;
receiving medical information from the first user over a network;
storing the medical information in the database;
associating the stored medical information with a barcode of the first user;
receiving data from a second user via a second user computing device sufficient to determine that the barcode of the first user has been scanned by the second user computing device; and
sending at least a portion of the stored medical information to the second user computing device over a network.

2. The method of claim 1, wherein the medical information comprises at least one of an existing medical condition of the first user,
an emergency treatment plan for the first user and emergency contact information for one or more emergency contacts associated with the first user.

3. The method of claim 2, wherein the medical information comprises emergency contact information for one or more emergency contacts associated with the first user, further comprising automatically sending a notification to a computing device of the one or more emergency contacts over a network using the emergency contact information, in particular wherein the notification comprises at least one of an email, text message, and a voice message, wherein the one or more emergency contacts comprises one or more personal contacts of the first user.

4. The method of one of the preceding claims, wherein the barcode is affixed to an article worn by the first user, or wherein the barcode is affixed to an article worn by an athlete during a sporting event.

5. The method of one of the preceding claims, wherein access to the database is provided through a web site or a software application.

6. The method of one of the preceding claims, further comprising receiving location information relating to the location of the second user computing device at the time the barcode was scanned thereby.

7. The method of one of the preceding claims, wherein the medical information comprises one or more of current diagnoses, emergency treatment plan, blood type, allergies, medical consent, prescriptions, physician information, emergency contacts, organ donor information, insurance information, and medical directive information.

8. The method of one of the preceding claims, further comprising receiving data from the second user computing device indicative of a characteristic of the second user, and sending at least a portion of the stored medical information to the second user computing device over the network dependent on the characteristic of the second user, in particular wherein the characteristic comprises the occupation of the second user and or the relationship of the second user with respect to the first user.

9. The method of one of the preceding claims, further comprising:
receiving data from a third user via a third user computing device sufficient to determine that the barcode of the first user has been scanned by the third user computing device; and
sending at least a portion of the stored medical information to the third user computing device over a network, wherein the stored medical information sent to the third user computing device is different from the stored medical information sent to the second user computing device.

10. The method of one of the preceding claims, further comprising receiving information from the user pertaining to at least one of appointment information and medication information, and sending a notification to the first user computing device based on the at least one of the appointment information and medication information.

11. A computer-implemented method for providing a medical alert system, the method comprising:
providing the first user with access to a database via a first user computing device;
receiving medical information from the first user over a network;
storing the medical information in the database;
providing a software application for the first user computing device for execution thereon, the software application comprising an activation input interface;
receiving an indication that the activation input interface has been activated over a network; and
sending at least a portion of the stored medical information to the first user computing device over a network in response to receiving the indication that the activation input interface has been activated, in particular
wherein the activation input interface comprises an icon on a display of the first user computing device.

12. A method of monitoring the health of a user, comprising:
receiving medical data at a user computing device from a medical device operative to communicate with the user computing device, the medical device being operative to monitor the user and to collect medical data relating to the user;
sending the medical data from the user computing device to a remote computing device over a network;
storing the medical data on the remote computing device;
analyzing the received medical data to identify medical information about the user; and
sending notification data to the user computing device dependent on the result of analyzing the received medical data.

13. The method of claim 12, wherein the medical device comprises one of a blood pressure cuff, glucose reader, pulse oximeter, and a weight measurement scale.

14. The method of claim 12 or 13, further comprising sending notification data to one or more computing devices associated with one or more individuals designated by the user.

15. A method of locating an animal, comprising:
providing a first user associated with the animal with access to a database via a first user computing device over a network;
receiving information from the first user over the network and storing the information in the database, the information being related to the animal;
associating the stored information with a barcode operative to be affixed to an article of the animal;
receiving data from a second user via a second user computing device sufficient to determine that the barcode has been scanned; and
sending at least a portion of the stored information to the second user computing device over a network.

16. The method of claim 15, wherein the information comprises contact information for the first user or another person associated with the animal, and or wherein the notification comprises the location of the second user computing device.

17. The method of claim 15 or 16, further comprising automatically sending a notification over a network using the stored information after determining that the barcode has been scanned.

18. A medical alert system comprising:
a server computing device;
a database communicatively coupled to the server computing device configured to store medical information thereon;
wherein the server computing device is operative to:
provide a first user with access to the database via a first user computing device;
receive medical information from the first user over a network and store the medical information in the database;
associate the stored medical information with a barcode of the first user;
receive data from a second user via a second user computing device sufficient to determine that the barcode of the first user has been scanned; and
send at least a portion of the stored medical information to the second user computing device over a network.

19. A non-transient computer-readable medium having instructions thereon for executing a process comprising:
providing a first user with access to a database via a first user computing device over a network;
receiving medical information from the first user over the network;
storing the medical information in the database;
associating the stored medical information with a barcode of the first user;
receiving data from a second user via a second user computing device sufficient to determine that the barcode of the first user has been scanned by the second user computing device; and
sending at least a portion of the stored medical information to the second user computing device over a network.
